Europäisches Patentamt

European Patent Office

Office européen des brevets

(11) Numéro de publication: **0 214 947**
**A1**

## (12) DEMANDE DE BREVET EUROPEEN

(21) Numéro de dépôt: 86870092.3

(22) Date de dépôt: 24.06.86

(51) Int. Cl.⁴: **A 61 K 31/65**
**//(A61K31/65, 31:595)**

(30) Priorité: 15.07.85 LU 86004

(43) Date de publication de la demande:
18.03.87 Bulletin 87/12

(84) Etats contractants désignés:
BE CH DE FR GB IT LI LU NL SE

(71) Demandeur: **S.S.M. INTERNATIONAL CHEMICAL COMPANY LIMITED**
**Lot 18**
**Kingstown(VC)**

(72) Inventeur: **Speecke, André**
**Rue Bahoux, 81**
**B-6680 Manhay(BE)**

(74) Mandataire: **De Brabanter, Maurice et al,**
**Bureau VANDER HAEGHEN 63 Avenue de la Toison d'Or**
**B-1060 Bruxelles(BE)**

(54) Composition antalgique et anti-inflammatoire destinée au traitement de l'arthrose.

(57) Il est possible de guérir l'arthrose à l'aide d'une composition antibiotique éventuellement injectable, constituée d'un mélange synergique d'un composé d'oxytétracycline et d'un complexe vitaminique A-$D_2$, complété éventuellement par de la vitamine B. Cette composition est exempte de cortisone.

EP 0 214 947 A1

Croydon Printing Company Ltd.

La présente invention est relative à une composition antibiotique éventuellement injectable, constituée d'un mélange synergique d'un composé d'oxytétracycline et d'un autre constituant.

Elle est destinée, en médecine humaine et éventuellement vétérinaire, à guérir l'arthrose et toutes les lésions dégénératives des articulations, ainsi que les synovites et particulièrement les inflammations du ménisque du genou.

Les lésions arthrosiques sont des inflammations chroniques des articulations dues à une prolifération des tissus osseux sous-jacents.

Les localisations les plus fréquentes des lésions arthrosiques sont les articulations intervertébrales, coxo-fémorales, les genoux et certaines articulations de la main et du pied.

Le symptôme essentiel est la douleur. Celle-ci est calmée par le repos mais exacerbée par les mouvements. Au stade initial, l'arthrose entraîne une limitation des mouvements et des craquements à la mobilisation. Elle fait ainsi apparaître des joints douloureux à la pression. A un stade avancé, on remarque des gonflements et même des déformations irréversibles des articulations.

Le traitement médical de l'arthrose envisage principalement l'administration de médications antalgiques et la prescription de médicaments anti-inflammatoires.

Entre autres, les corticoïdes sont sans conteste efficaces et calment immédiatement l'accès aigu des douleurs, mais ils présentent aussi de graves inconvénients. Ils entraînent en effet des risque infectieux qui rendent parfois nécessaire une association d'antibiotiques et des risques digestifs, rénaux et neurologiques, du fait de leur grande toxicité.

La présente invention vise à traiter l'arthrose sans présenter les inconvénients de la corticothérapie. Elle est relative à une composition médicamenteuse, permet un traitement de fond, précoce et prolongé de l'arthrose et prévient toute récidive.

Cette composition médicamenteuse peut être administrée selon sa forme galénique, par voie orale ou parentérale. Elle concerne un mélange synergique d'un antibiotique du type oxytétracycline et d'un autre constituant, essentiellement caractérisé en ce que cet autre constituant est un complexe vitaminique A-D.

Suivant une particularité de l'invention, l'oxytétracycline et le complexe vitamine A-D sont mélangés dans un rapport pondéral de 0,3 à 3.

L'activité de la composition susdite est avantageusement renforcée par la présence d'un complexe de vitamines B, contenant en particulier de la vitamine $B_1$ ''antinévritique'', de la vitamine $B_6$ ''antidermatosique'' et de la vitamine PP (ou $B_3$) ''antipellagreuse''.

3

D'autres particularités et détails de l'invention apparaîtront au cours de la description détaillée donnée ci-dessus, d'une forme galénique particulière de l'invention.

L'oxytétracycline est un composé antibiotique que l'on isole des substances élaborées par les actinomycètes et en particulier du Streptomyces rimosus. Elle a été décrite pour la première fois dans le brevet américain N° 2 516 080 (Pfizer). Elle est principalement utilisée comme anti-bactérien contre certaines maladies infectieuses. Elle est administrée en particulier pour combattre les bactéries du groupe des Salmonella et des Pseudomonas.

L'adjonction d'autres antibiotiques, de Pénicilline G et de sulfate de colistine suivant le brevet belge N° 505 709 et la demande de brevet luxembourgeois N° 84 786 élargit de façon remarquable les spectres d'applicabilité de l'oxytétracycline pour combattre le bacille de Koch et les bactéries du groupe des Salmonella et des Pseudomonas, responsables de maladies dangereuses pour l'homme et la volaille. Notons pour l'homme, la fièvre paratyphoïde et l'entérocolite salmonellique. L'oxytétracycline est caractérisée par un spectre extrêmement large et une toxicité réduite.

La présente invention élargit le champ d'application de l'oxytétracycline et étend celui-ci au traitement précoce et néanmoins radical de maladies et traumatismes des articulations.

L'invention est basée sur la découverte que l'oxytétracycline ou Terramycine présente une efficacité étonnante contre l'arthrose lorsqu'elle est administrée en même temps qu'un complexe vitaminé A-D$_2$. D'excellents résultats sont obtenus lorsque les composants sont mélangés dans un rapport pondéral variant de 0,3 à 5.

Les vitamines A-D$_2$ sont destinées principalement à fixer le calcium.

Cette synergie de l'oxytétracycline et d'un complexe vitaminé A-D$_2$ est renforcée par la présence de quantité d'un complexe B destiné à pallier les troubles digestifs éventuels.

Les vitamines du complexe B comprenant les vitamines B$_1$, B$_2$, PP, B$_6$, B$_{12}$, l'acide pantothénique et la biotine entrent dans la composition des différents systèmes fermentaires qui régularisent le métabolisme général et permet de rétablir rapidement la fonction normale de la flore intestinale après une perturbation.

On remarque donc que les indications médicales premières de chacun des constituants décrites ci-dessus, pris séparément, sont totalement différentes de celles de leur mélange.

La composition synergique suivant l'invention trouve son application préférée dans le traitement de l'arthrose. Elle permet de guérir les lésions dégénératives des articulations. Ses effets antalgiques et anti-inflammatoires sont immédiats. Elle permet de traiter efficacement les synovites, les épanchements de synovie et les autres inflammations du ménisque du genou,

5

fréquents chez les footballeurs professionnels.

Elle procure des résultats spectaculaires dans le traitement des sciatiques, rhumatismes, décalcifications des os, hernies discales, lombagos, entorses et déchirures musculaires.

L'exemple suivant de composition est destiné à illustrer le caractère nouveau et inventif de la composition médicamenteuse. Ni la composition précise, ni la forme galénique ne doit être considérée comme une limitation. L'analyse suivante détermine les quantités de chacun des constituants présents dans une cuillerée à café, c'est-à-dire 5 ml de la composition suivant l'invention.

| Constituants | Quantité par 5 ml |
|---|---|
| 1/ Chlorhydrate d'oxytétracycline (calculée sur base d'oxytétracycline-base) | 50-150 mg |
| 2/ Vitamine A (Ergocalciférol) | 50.000-150.000 U.I. |
| 3/ Vitamine $D_2$ (Hydroxytoluène butyle) | 50.000-150.000 U.I. |
| 4/ Vitamine $B_1$ (chlorhydrate de thiamine ou Aneurine) | 1,5-3 mg |
| 5/ Vitamine $B_2$ (Riboflavine) | 400-650 µg |
| 6/ Vitamine $B_6$ (Pyridoxine) | 200-450 µg |
| 7/ Vitamine $B_{12}$ (Cyanocobalamine) | néant |
| 8/ Vitamine PP (Nicotinamide) | 3-10 mg |
| 9/ Vitamine $B_5$ (Pantothénate de calcium) | néant |
| 10/ Dexpanthénol | 40-80 µg |
| 11/ Biotine | néant |
| 12/ Oxyde de magnésium | 100-300 µg |
| 13/ Sirop de sucre | ad 5 ml |

EXEMPLE 1

Une forme préférée d'exécution présente une composition répondant à la formule suivante, contenant par 5 ml, les constituants suivants :

| Constituants | Par cuillerée à café de 5 ml |
|---|---|
| 1/ Chlorhydrate d'oxytétracycline (calculée sur base d'oxytétracycline-base) | 70 mg |
| 2/ Vitamine A (Ergocalciférol) | 115.000 U.I. |
| 3/ Vitamine $D_2$ (Hydroxytoluène-butyle) | 115.000 U.I. |
| 4/ Vitamine $B_1$ (chlorhydrate de thiamine ou Aneurine) | 2,2 mg |
| 5/ Vitamine $B_2$ (Riboflavine) | 530 µg |
| 6/ Vitamine $B_6$ (Piridoxine) | 300 µg |
| 7/ Vitamine $B_{12}$ (Cyanocobalamine) | néant |
| 8/ Vitamine PP (Nicotinamide) | 6 mg |
| 9/ Vitamine $B_5$ (Pantothénate de calcium) | néant |
| 10/ Dexpanthénol | 60 µg |
| 11/ Biotine | néant |
| 12/ Oxyde de magnésium | 175 µg |
| 13/ Sirop de sucre | ad 5 ml |

EXEMPLE 2

Une autre formule possible d'exécution présente une composition répondant à la formule suivante, contenant par 5 ml les constituants suivants donnés en milligrammes:

**0214947**

| Constituants | Par cuillerée de 5 ml |
|---|---|
| Chlorhydrate d'oxytétracycline | 70 mg |
| Vit. A | 50.000 U.I. |
| Vit. $D_2$ | 50.000 U.I. |
| Vit. $B_1$ | 1,425 mg |
| Vit. $B_2$ | 0,225 mg |
| Vit. $B_6$ | 0,225 mg |
| D-Pantothénol | 0,225 mg |
| Vit. PP | 3 mg |
| Excipients | ad 5 ml |

Le mélange de l'exemple 1 est réalisé en mélangeant entre eux 100 ml d'une solution injectable d'oxytétracycline à 10 %, contenant par exemple 92,6 mg de chlorhydrate d'oxytétracycline par ml de solution, 150 mg d'un complexe vitaminique A-D, commercialisé par exemple par Pfizer pour usage vétérinaire. On ajoute ensuite 100 ml de sirop de complexes vitaminiques B, par exemple de BECOZYME® Roche (sirop) et/ou de BECOVITAN® (sirop, produit par Cilag N.U./S.A. et environ 200 ml d'un sirop de sucre, de manière à obtenir 650 ml de composition médicamenteuse sous forme d'un sirop.

La préparation sous forme de sirop aux dosages indiqués ci-dessus est administrée par voie buccale à raison de trois cuillerées à café pendant 3 jours. Le traitement est poursuivi par la prise d'une cuillerée à café pendant quelques jours.

Les contre-indications sont déterminées par les hypercalcémies qui peuvent entraîner un blocage de reins. La composition doit être prise sous contrôle médical.

Il convient de noter qu'en variante, chacun des constituants peut être ajouté sous forme injectable, en particulier le complexe vitaminé BECOZYME® Roche et/ou BECOVITAN forte® de manière à obtenir une solution huileuse injectable. Cette forme galénique favorise l'assimilation de la vitamine $B_{12}$ et de la biotine.

D'autres modifications peuvent être apportées à la forme de réalisation de l'invention. Il est évident que l'homme de métier peut apporter de nombreuses variantes dans le choix, notamment des excipients, supports ou diluants. En principe donc, l'invention couvre toutes les combinaisons synergiques de l'invention avec les supports, diluants et excipients utilisés habituellement en pharmacologie.

<u>REVENDICATIONS</u>

1. Composition antibiotique comprenant, éventuellement sous forme injectable, un mélange synergique d'un antibiotique du type oxytétracycline et d'un autre constituant, caractérisé en ce que cet autre constituant est un complexe vitaminique $A-D_2$.

2. Composition selon la revendication 1, caractérisé en ce que l'oxytétracycline et le complexe vitaminé $A-D_2$ sont mélangés dans un rapport pondéral de 0,3 à 3.

3. Composition selon l'une quelconque des revendications 1 et 2, caractérisée en ce qu'elle comprend également un complexe vitaminique B.

4. Composition selon l'une quelconque des revendications 1 à 3, caractérisée en ce qu'elle correspond à la formule galénique obtenue en mélangeant pour 5 ml (1 cuillerée à café) les constituants suivants :

| Constituants | Quantité par 5 ml |
|---|---|
| 1/ Chlorhydrate d'oxytétracycline (calculée sur base d'oxytétra-cycline-base) | 50-150 mg |
| 2/ Vitamine A (Ergocalciférol) | 50.000-150.000 U.I. |
| 3/ Vitamine $D_2$ (Hydroxytoluène butyle) | 50.000-150.000 U.I. |
| 4/ Vitamine $B_1$ (chlorhydrate de thiamine ou Aneurine) | 1,5-3 mg |
| 5/ Vitamine $B_2$ (Riboflavine) | 400-650 µg |
| 6/ Vitamine $B_6$ (Pyridoxine) | 200-450 µg |
| 7/ Vitamine $B_{12}$ (Cyanocobalamine) | néant |
| 8/ Vitamine PP (Nicotinamide) | 3-10 mg |
| 9/ Vitamine $B_5$ (Pantothénate de calcium) | néant |
| 10/ Dexpanthénol | 40-80 µg |
| 11/ Biotine | néant |
| 12/ Oxyde de magnésium | 100-300 µg |
| 13/ Sirop de sucre | ad 5 ml |

5. Composition selon la revendication 4, caractérisée en ce qu'elle répond à l'analyse suivante :

| Constituants | Par cuillerée à café de 5 ml |
|---|---|
| 1/ Chlorhydrate d'oxytétracycline (calculée sur base d'oxytétra- cycline-base) | 70 mg |
| 2/ Vitamine A (Ergocalciférol) | 115.000 U.I. |
| 3/ Vitamine $D_2$ (Hydroxytoluène-butyle) | 115.000 U.I. |
| 4/ Vitamine $B_1$ (chlorhydrate de thiamine ou Aneurine) | 2,2 mg |
| 5/ Vitamine $B_2$ (Riboflavine) | 530 µg |
| 6/ Vitamine $B_6$ (Piridoxine) | 300 µg |
| 7/ Vitamine $B_{12}$ (Cyanocobalamine) | néant |
| 8/ Vitamine PP (Nicotinamide) | 6 mg |
| 9/ Vitamine $B_5$ (Pantothénate de calcium) | néant |
| 10/ Dexpanthénol | 60 µg |
| 11/ Biotine | néant |
| 12/ Oxyde de magnésium | 175 µg |
| 13/ Sirop de sucre | ad 5 ml |

6. Utilisation d'une composition selon l'une des revendications 1 à 5, pour traiter l'arthrose, les synovies, les épanchements de synovie, les inflammations du ménisque du genou, les sciatiques, les rhumatismes, les décalcifications des os, les hernies discales, les lumbagos, les entorses et déchirures musculaires.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | UNLISTED DRUGS, vol. 24, no. 10, octobre 1972, Chatham, New Jersey, US; * Page 158b, "Sanoxy" * | 1-6 | A 61 K 31/65 // (A 61 K 31/65 A 61 K 31:595) |
| | --- | | |
| A | UNLISTED DRUGS, vol. 25, no. 11, novembre 1973, Chatham, New Jersey, US; * Page 180g, "Vet-rex" * | 1-6 | |
| | ----- | | |

**DOMAINES TECHNIQUES RECHERCHES (Int. Cl. 4)**

A 61 K

Le présent rapport de recherche a été établi pour toutes les revendications

| Lieu de la recherche | Date d'achevement de la recherche | Examinateur |
|---|---|---|
| LA HAYE | 24-10-1986 | BRINKMANN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& – membre de la même famille, document correspondant

OEB Form 1503 03 82